# FASCICULE DE BREVET EUROPEEN

(11) **EP 1 018 322 B1**
(45) Date de publication et mention de la délivrance du brevet: **12.03.2008**
(21) Numéro de dépôt: 99403314.0
(22) Date de dépôt: 29.12.1999
(51) Int. Cl.: A61F 2/38

(54) **Prothèse tibiale du genou à double insert**
Tibiale Knieprothese mit doppeltem Einsatz
Tibial knee prosthesis with a double insert

(30) Priorité: 04.01.1999 FR 9900012
(43) Date de publication de la demande: 12.07.2000
(73) Titulaire: Aesculap Société par actions simplifiée, 52000 Chaumont (FR); Reigner, Bernard, Dr., 72000 Le Mans (FR)
(72) Inventeur: Reigner, Bernard, Dr., 72000 Le Mans (FR); Biegun, Jean-François, 52000 Chuamont (FR); Marceaux, Pascal, 52000 Chaumont (FR)
(74) Mandataire: Eidelsberg, Victor Albert

(56) Documents cités:
- EP-A- 0 724 868
- DE-A- 3 529 894
- FR-A- 2 776 919
- US-A- 4 268 920

## Description

La présente invention concerne une endoprothèse du genou, du genre comportant une partie tibiale constituée d'une tige tibiale, destinée à être insérée par une extrémité dans le tibia du patient, et d'une plaque tibiale fixée à l'autre extrémité de la tige tibiale ; un insert tibial disposé sur la plaque tibiale ; et une partie fémorale constituée d'une tige fémorale et de condyles portés par la tige fémorale comportant chacun une première surface de glissement de forme correspondante à une seconde surface de glissement de l'insert tibial, les condyles étant séparés au moins partiellement par un intervalle intercondylien ;

les condyles et l'insert tibial coopérant par l'intermédiaire d'un organe de couplage, constitués par exemple d'un tourillon qui passe à travers l'insert tibial par une ouverture formée dans l'insert et qui est fixé à la partie tibiale ; d'une tête de couplage, tête de couplage qui pénètre dans l'intervalle intercondylien et d'un axe de couplage, fixé aux condyles et pénétrant dans une ouverture de la tête de couplage, la partie fémorale pouvant ainsi pivoter par rapport à cet axe de couplage de manière à pouvoir passer d'une position déployée (extension totale du genou) à une position pliée (flexion à environ 120° du genou).

Une prothèse du genou de ce genre est connue par exemple du document EP-0.791.343.

Les prothèses du genou de ce genre présentent plusieurs inconvénients.

L'insert tibial, en général en polyéthylène, est monté libre en rotation par rapport à la plaque tibiale (généralement métallique) de manière à permettre un jeu en rotation propre du fémur par rapport au tibia, cette rotation propre s'effectuant par l'intermédiaire des condyles (en général métalliques) glissant sur les surfaces de glissement correspondantes de l'insert tibial. En position déployée (angle α de flexion du genou = 0°) du genou, la rotation du fémur par rapport au tibia est bloquée par l'intermédiaire d'une liaison en butée des condyles sur l'insert en polyéthylène. Cependant, dès que le genou vient à être plié (α devient supérieur à 0°), ce blocage est totalement relâché et le fémur peut donc subir des déplacements en rotation axiale très grands alors que le genou est à peine plié. Cela ne reproduit pas la réalité du genou anatomique et peut entraîner des coincements que l'on ne souhaite pas.

L'invention vise à pallier ces inconvénients en proposant une prothèse dans laquelle le verrouillage de la partie fémorale à la partie tibiale, lors du déplacement du genou jusqu'à une position totalement déployée du genou, s'effectue progressivement au cours de l'extension (déploiement) du genou.

Le document DE-A-3529894 décrit une prothèse dont l'insert tibial présente un élément de blocage progressif destiné à limiter le déplacement en rotation propre des condyles par rapport à la plaque tibiale à partir d'un angle α₀ de flexion du genou déterminé à l'avance jusqu'à un angle nul, l'amplitude de déplacement en rotation propre allant en diminuant de cet angle α₀ jusqu'à une amplitude nulle (blocage total) pour l'angle nul (genou déplié).

Suivant l'invention, cet élément de blocage progressif est constitué d'un second insert tibial monté fixe sur la plaque tibiale et dont la forme est telle que, en position étendue du genou, le second insert tibial fixe vient faire saillie dans un intervalle intercondylien formé entre les condyles et s'insère dans le siège formé par l'intervalle intercondylien de manière à bloquer toute rotation mutuelle latérale ou axiale du fémur par rapport au tibia et telle que lors de la sortie du second insert de son siège lors de la flexion du genou, il s'instaure entre les parois de l'intervalle intercondylien et le second insert un jeu progressif jusqu'à un angle de flexion (α₀) déterminé à l'avance, à partir duquel les déplacements en rotation axiale sont libres.

Suivant cet agencement particulier, on évite que l'insert tibial rotatif serve au blocage progressif des condyles et donc s'use aussi rapidement que dans le cas où l'élément de blocage fait partie intégrante de l'insert tibial, auquel cas l'insert tibial, qui est libre en rotation par rapport à la plaque tibiale subit des contraintes aussi bien dues à la flexion qu'aux rotations axiales ou en varus/valgus. Maintenant, l'insert mobile ne subit plus de contrainte qu'en flexion, les contraintes en rotation axiale ou en varus/valgus, notamment en position bloquée, étant subies entièrement par le second insert tibial fixe.

Suivant un perfectionnement de l'invention, on prévoit de rendre fixe l'organe de couplage par rapport à la plaque tibiale pour limiter encore plus les possibilités de rotation propre, et ainsi encore mieux bloquer en position déployée le fémur par rapport au tibia.

La figure 1 est une vue en coupe latérale d'une prothèse de genou suivant l'invention, en position déployée.

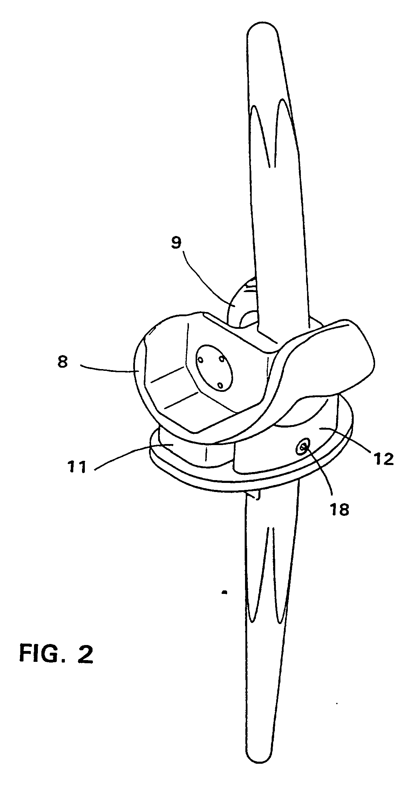

La figure 2 est une vue en perspective de la prothèse de la figure 1, également en position déployée.

La figure 3 est une vue en perspective de la prothèse de la figure 2, en position pliée.

Les figures 4a, 4b et 4c sont des vues, respectivement, de côté, de côté avant et en coupe A-A de l'insert fixe.

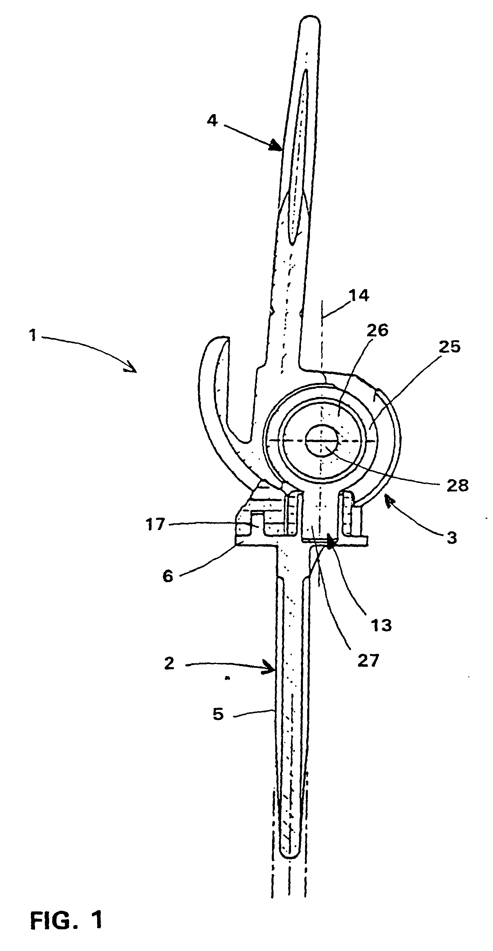

A la figure 1, il est représenté une prothèse 1 du genou, constituée d'une partie 2 tibiale, d'une partie 3 de couplage et d'une partie 4 fémorale.

La partie 2 tibiale comprend une tige 5 tibiale destinée à être insérée et fixée dans le canal médullaire du tibia et une plaque 6 tibiale portée par la tige 5 tibiale.

La partie 4 fémorale comprend une tige 7 fémorale destinée à être insérée et fixée dans le fémur du patient et deux condyles 8, 9 de forme concave, les deux condyles 8, 9 étant séparés par un intervalle.

La partie 3 de couplage est constituée d'une part d'un premier insert 11 tibial, d'un deuxième insert 12 tibial et d'un organe 13 de couplage.

Le premier insert 11 tibial, qui est par exemple en polyéthylène est disposé sur la plaque 6 tibiale de manière à être libre en rotation par rapport à un premier axe de rotation 14, sensiblement parallèle à l'axe longitudinal de la tige 5 tibiale et légèrement décalé vers le côté antérieur du genou, par rapport à la tige 5 tibiale. Le premier insert 11 tibial, ou insert rotatif, comporte sur sa partie supérieure deux cavités dont les surfaces extérieures correspondent, de manière congruente, aux surfaces extérieures des condyles 8, 9.

Le deuxième insert 12 tibial, également par exemple en polyéthylène, est monté fixe sur la plaque 6 tibiale, une saillie mâle 17 de la plaque tibiale étant introduite dans une cavité femelle formée dans la partie inférieure de l'insert 12 fixe, de manière à fixer l'insert 12 fixe vis-à-vis de la plaque 6 tibiale. En outre une vis 18 fixe par vissage l'insert à la saillie mâle 17.

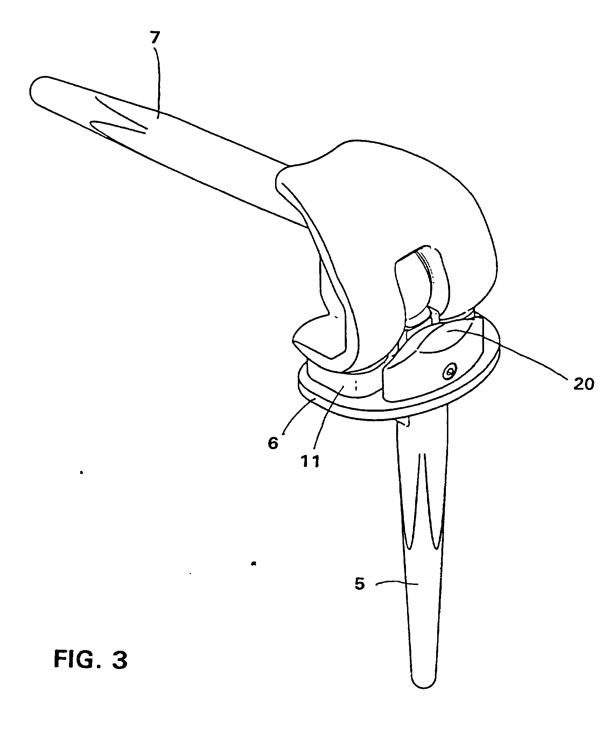

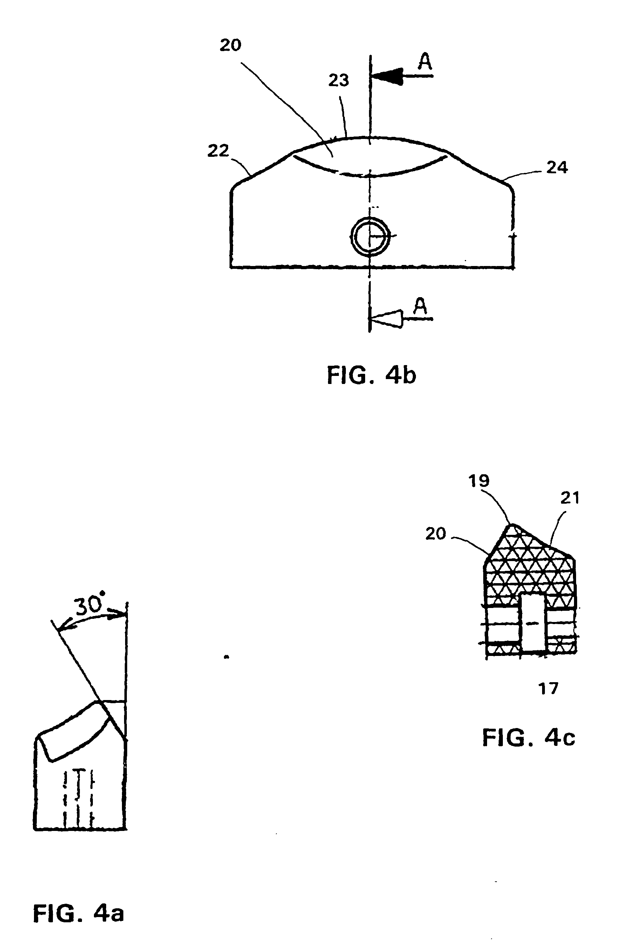

L'insert 12 fixe est mieux représenté aux figures 4a, 4b et 4c. Il comporte sur sa partie supérieure une crête 19 séparant la face supérieure en deux surfaces 20, 21 supérieures, qui sont chacune inclinées vers le bas en sens opposé. La crête 19 comporte trois tronçons 22, 23, 24 sensiblement en forme d'arcs de cercle dont les convexités sont altérnées, le tronçon de milieu 23 ayant sa convexité tournée vers le haut, tandis que les deux autres tronçons 22, 24 latéraux de la crête ont leurs convexités tournées vers le bas (de la figure). La surface supérieure 21 tournée vers l'insert 11 mobile a une forme incurvée symétrique, par rapport au plan des deux tiges 5 et 7 tibiale et fémorale à la figure 3, de manière à former deux surfaces de glissement pour les surfaces de glissement des condyles.

L'organe 13 de couplage est constitué d'un tourillon 27 qui est monté, soit fixe en rotation, en ayant par exemple une section transversale du type 6-pans (non représentée aux figures) qui coopère avec les parois latérales de section transversale correspondante d'un évidement dans la tige 6 tibiale, soit rotatif dans un évidement de cette même tige 6 tibiale ayant par exemple une paroi latérale cylindrique circulaire. Le tourillon 19 traverse l'insert rotatif par une ouverture centrale de l'insert 11 rotatif, de sorte que le tourillon 27 forme l'axe de rotation de l'insert 11 rotatif.

L'organe 13 de couplage comprend également une tête 25 de couplage qui pénètre dans l'intervalle intercondylaire. Cette tête 25 de couplage comprend un évidemment 26 dans lequel pénètre un axe 28 de couplage, fixé de part et d'autre de l'intervalle intercondylien aux condyles 8, 9, l'axe de couplage transversalement perpendiculaire à l'axe de rotation de l'insert 11 rotatif et éventuellement du tourillon 19 est perpendiculaire à l'axe antéro-postérieur.

La surface extérieure de l'axe 28 de couplage a, dans la partie se trouvant dans la tête de couplage, une forme sphérique et coopère avec une forme sphérique de l'évidemment de la tête de couplage.

La partie 4 fémorale pivote, en flexion, par rapport à la partie 2 tibiale, suivant l'axe 28 de couplage, pour passer d'une position où le genou est étendu (fémur et tibia sensiblement parallèles, α = 0) à une position où le genou est fléchi au maximum (fémur et tibia formant un angle α ≈ 120°.

En position étendue, les surfaces extérieures des condyles 8,9 viennent en butée sur la crête 19 de l'insert fixe qui l'immobilise, d'une part en rotation de flexion, en empêchant la poursuite de la flexion et d'autre part également en rotation axiale, la surface extérieure concaves des deux condyles 8, 9 du côté avant du genou recevant entre elles la partie en forme de crête de l'insert 12 fixe et venant buter contre elles dans leur mouvement en rotation axiale.

Au fur et à mesure que le genou se plie, les surfaces extérieures des condyles 8, 9 du côté avant du genou, quittent leur coopération avec la crête 19 et la surface 21 extérieure, de sorte qu'un jeu progressif apparaît entre chaque condyle et sa partie de surface respective de la surface 21 extérieure. Ainsi le fémur peut progressivement avoir de nouveau un mouvement en rotation axiale. La forme des condyles et celle de la crête de l'insert fixe sont telles que la rotation axiale du fémur n'est libérée entièrement qu'au-delà d'un certain angle de flexion, par exemple ici 30°. Avant d'atteindre cet angle de flexion, la rotation axiale est limitée dans un domaine déterminée par les formes respectives de la crête et de la surface 21 extérieure, par exemple ici ± 10° pour α₀ = 30°.

On définit l'angle α comme étant l'angle formé entre l'axe longitudinal du fémur et celui du tibia, α pouvant varier de 0 (fémur et tibial parallèles, genou étendu) à 120° (angle maximal de flexion du genou).

## Revendications

1. Endoprothèse (1) du genou, comportant :
- une partie (2) tibiale, constituée d'une tige (5) tibiale portant une plaque (6) tibiale,
- un premier insert (11) tibial disposé sur la plaque (6) tibiale,
- une partie (4) fémorale, constituée d'une tige (7) fémorale, qui porte deux condyles (8, 9) ayant chacun une première surface de glissement coopérant, de manière congruente, avec une deuxième surface de glissement respective formée sur le premier insert (11) tibial,
- un organe (3) de couplage destiné à coupler la partie (2) tibiale à la partie (4) fémorale pour permettre une flexion du fémur par rapport au tibia d'une position (α sensiblement = 0) où le genou est étendu à une position (αₘₐₓ) où le genou est plié et inversement, et
- un élément de blocage progressif destiné à limiter le déplacement en rotation latérale et en rotation propre des condyles (8, 9) par rapport à la plaque tibiale à partir d'un angle α₀ de flexion du genou déterminé à l'avance jusqu'à un angle nul, l'amplitude de déplacement en rotation propre allant en diminuant de cet angle α₀ jusqu'à une amplitude nulle (blocage total) pour l'angle nul (genou déplié) ;
**caractérisée en ce que** l'élément de blocage progressif est constitué d'un second insert (12) tibial monté fixe sur la plaque tibiale et dont la forme est telle que, en position étendue du genou, le second insert tibial fixe vient faire saillie dans un intervalle intercondylien formé entre les condyles (8, 9) et s'insère dans le siège formé par l'intervalle intercondylien de manière à bloquer toute rotation axiale du fémur par rapport au tibia et telle que lors de la sortie du second insert de son siège lors de la flexion du genou, il s'instaure entre les parois de l'intervalle intercondylien et le second insert un jeu progressif jusqu'à l'angle de flexion (α₀) déterminé à l'avance, à partir duquel les déplacements en rotation axiale sont libres.

2. Endoprothèse suivant la revendication 1, **caractérisée en ce que** le second insert (12) tibial a une surface supérieure comportant une crête (19) et une surface (21) extérieure, et la forme des condyles (8, 9) du côté avant du genou sont telles, qu'en position étendue du genou, la crête (19) bloque en flexion, comme en rotation propre tout mouvement du fémur et qu'au fur et à mesure que le genou se plie, un jeu progressif apparaît entre les condyles et la crête (19) et la surface (21) extérieure, pour permettre une possibilité limitée en rotation propre jusqu'à un angle de flexion déterminé à l'avance et une libération totale au delà de l'angle de flexion déterminé à l'avance.

3. Endoprothèse suivant la revendication 1 ou 2 dans laquelle le premier insert (11) est monté rotatif par rapport à la plaque tibiale.

4. Endoprothèse suivant l'une des revendications 1 à 3, dans laquelle l'angle α₀ déterminé à l'avance est compris entre 20 et 40°, de préférence 30°.

5. Endoprothèse suivant l'une des revendications 2 à 4, **caractérisée en ce que** la crête (19) de l'insert (12) est constituée de trois tronçons (22, 23, 24) en forme d'arcs de cercle dont les convexités sont alternées, le tronçon du milieu (23) ayant sa convexité tournée vers le haut de manière à pouvoir pénétrer dans l'intervalle intercondylien, et **en ce que** la surface extérieure (21) vers l'insert (11) mobile a une forme incurvée pour former deux deuxièmes surfaces de glissement respectives pour les premières surfaces extérieures de glissement des condyles (8, 9).

6. Endoprothèse suivant l'une des revendications 1 à 5, dans laquelle l'organe de couplage est constitué d'un tourillon (27) et d'une tête (25) de couplage, le tourillon étant monté fixe en rotation ou rotatif dans la tige (5) tibiale, en traversant le premier insert (11) par une ouverture de l'insert, la tête (25) de couplage étant évidée pour recevoir un axe (28) de couplage fixé aux condyles et assurant la rotation en flexion du fémur par rapport au tibia.

## Claims

1. A knee endoprosthesis (1) comprising:
- a tibial part (2) comprising a tibial rod (5) bearing a tibial plate (6),
- a first tibial insert (11) disposed on the tibial plate (6),
- a femoral part (4) comprising a femoral rod (7) bearing two condyles (8, 9) each having a first sliding surface co-operating and congruent with a respective second sliding surface formed on the first tibial insert (11),
- a coupling means (3) for coupling the tibial part (2) to the femoral part (4) so that the femur can bend relative to the tibia from a position (α ≈ 0) where the knee is extended to a position (α ₘₐₓ) where the knee is bent and vice-versa, and
- a progressive locking element for limiting the lateral rotation and proper rotation of the condyles (8, 9) relative to the tibial plate, starting from a knee-bending angle α₀ determined in advance and up to a zero angle, the amplitude of proper rotation, when the angle α₀ is decreased, varying down to zero amplitude (complete blocking) at zero angle (unbent knee) ;
**characterised in that** the progressive locking element comprises a second tibial insert (12) fixed to the tibial plate and having a shape such that when the knee is in the extended position the second fixed tibial insert projects into an intercondylar space formed between the condyles (8, 9) and fits into the seat formed by the intercondylar space so as to block any axial rotation of the femur relative to the tibia, and such that when the knee bends and the second insert comes out of its seat, a clearance between the walls of the intercondylar space and the second insert occurs progressively up to a bending angle (α₀) determined in advance, after which the movements in axial rotation are free.

2. An endoprosthesis according to claim 1, **characterised in that** the second tibial insert (12) has an upper surface comprising a ridge (19) and an outer surface (21), and the shape of the condyles (8, 9) on the front of the knee are such that when the knee is in the extended position the ridge (19) blocks any bending or proper rotation of the femur whereas as the knee bends a progressive clearance occurs between the condyles and the ridge (19) and on outer surface (21), permitting limited proper rotation up to a bending angle determined in advance and complete freedom beyond the bending angle determined in advance.

3. An endoprosthesis according to claim 1 or 2 wherein the first insert (11) is mounted so as to be rotary relative to the tibial plate.

4. An endoprosthesis according to any of claims 1 to 3, wherein the angle α₀ determined in advance is between 20 and 40°, preferably 30°.

5. An endoprosthesis according to any of claims 2 to 4, **characterised in that** the ridge (19) on the insert (12) comprises three portions (22, 23, 24) in the form of arcs of a circle and having alternate convexity, the middle portion (23) having upward-turned convexity so that it can extend into the intercondylar space, and **in that** said outer surface (21) facing the movable insert (11) is curved so as to form two second sliding surfaces respectively for the first outer sliding surfaces on the condyles (8, 9).

6. An endoprosthesis according to any of claims 1 to 5, wherein the coupling means comprises a journal (27) and a coupling head (25), the journal being mounted so as to be fixed in rotation or rotary in the tibial rod (5) and extending through the first insert (11) via an opening therein, the coupling head (25) being recessed so as to receive a coupling shaft (28) fixed to the condyles and enabling the femur to bend by rotating relative to the tibia.

## Patentansprüche

1. Endoprothese (1) des Knies, mit:
- einem tibialen Abschnitt (2), der aus einem tibialen Schaft (5) gebildet ist, der eine tibiale Platte (6) trägt,
- einem ersten tibialen Einsatzstück (11), das an der tibialen Platte (6) angeordnet ist,
- einem femoralen Abschnitt (4), der aus einem femoralen Schaft (7) gebildet ist, welcher zwei Gelenkköpfe (8,9) trägt, die jeweils eine erste Gleitfläche aufweisen, die auf kongruente Weise mit einer jeweiligen an dem ersten tibialen Einsatzstück (11) ausgebildeten zweiten Gleitfläche zusammenwirkt,
- einem Kopplungselement (3), das dazu bestimmt ist, den tibialen Abschnitt (2) mit dem femoralen Abschnitt (4) zu koppeln, um eine Flexion des Femurs in bezug auf die Tibia aus einer Position (α im wesentlichen = 0), in der das Knie gestreckt ist, zu einer Position (αₘₐₓ), in der das Knie gebeugt ist, und umgekehrt zu ermöglichen, und
- einem Element zur progressiven Blockierung, das dazu bestimmt ist, die laterale Drehbewegung und die Eigendrehbewegung der Gelenkköpfe (8,9) in bezug auf die tibiale Platte von einem Flexionswinkel α₀ des Knies, der vorab festgelegt wird, bis zu einem Nullwinkel zu begrenzen, wobei die Bewegungsamplitude der Eigendrehung von diesem Winkel α₀ aus bis zu einer Nullamplitude (Totalblockierung) für den Nullwinkel (gestrecktes Knie) abnimmt,
**dadurch gekennzeichnet, dass** das progressive Blockierelement aus einem zweiten tibialen Einsatzstück (12) gebildet ist, das fest an der tibialen Platte angebracht ist und dessen Form derart ist, dass in einer gestreckten Position des Knies das zweite feststehende tibiale Einsatzstück in einem zwischen den Gelenkköpfen (8,9) ausgebildeten Zwischengelenkkopf-Intervall vorsteht und in den durch das Zwischengelenkkopf-Intervall gebildeten Sitz so eingefügt ist, dass es die gesamte axiale Drehung des Femurs in bezug auf die Tibia blockiert, und derart, dass beim Austreten des zweiten Einsatzstückes aus seinem Sitz bei der Biegung des Knies ein progressives Spiel zwischen den Wänden des Zwischengelenkkopf-Intervalls und dem zweiten Einsatzstück bis zu dem vorab festgelegten Flexionswinkel (α₀), ab dem die axialen Drehbewegungen frei sind, entsteht.

2. Endoprothese nach Anspruch 1, **dadurch gekennzeichnet, dass** das zweite tibiale Einsatzstück (12) eine obere Oberfläche mit einem Steg (19) und einer Außenfläche (21) hat, und dass die Form der Gelenkköpfe (8,9) auf der Vorderseite des Knies derart ist, dass in einer gestreckten Position des Knies der Steg (19) bei der Biegung wie bei der Eigendrehung jede Bewegung des Femurs blockiert, und dass im Verlauf der weiteren Biegung des Knies ein progressives Spiel zwischen den Gelenkköpfen und dem Steg (19) und der Außenfläche (21) in Erscheinung tritt, um eine begrenzte Eigendrehmöglichkeit bis zu einem vorab festgelegten Flexionswinkel sowie eine totale Freigabe jenseits des vorab festgelegten Flexionswinkels zu gestatten.

3. Endoprothese nach Anspruch 1 oder 2, wobei das erste Einsatzstück (11) drehbar in bezug auf die tibiale Platte angebracht ist.

4. Endoprothese nach einem der Ansprüche 1 bis 3, wobei der vorab festgelegte Winkel α₀ zwischen 20 und 40°, vorzugsweise bei 30° liegt.

5. Endoprothese nach einem der Ansprüche 2 bis 4, **dadurch gekennzeichnet, dass** der Steg (19) des Einsatzstücks (12) aus drei in Kreisbogenform ausgebildeten Teilstücken (22,23,24) gebildet ist, deren Konvexitäten alternieren, wobei die Konvexität des mittleren Teilstücks (23) nach oben gerichtet ist, so dass er in das Zwischengelenkkopf-Intervall ein dringen kann, und dass die Außenfläche (21), die dem beweglichen Einsatzstück (11) zugewandt ist, eine gekrümmte Form aufweist, um jeweils zwei zweite Gleitflächen für die ersten äußeren Gleitflächen der Gelenkköpfe (8,9) zu bilden.

6. Endoprothese nach einem der Ansprüche 1 bis 5, wobei das Koppelungselement aus einem Zapfen (27) und einem Koppelungskopf (25) gebildet ist, wobei der Zapfen drehfest oder drehbar in dem tibialen Schaft (5) angebracht ist, indem er das erste Einsatzstück (11) über eine Einführöffnung durchsetzt, und der Koppelungskopf (25) eine Ausnehmung aufweist, um eine an den Gelenkköpfen befestigte und die Biegedrehung des Femurs in bezug auf die Tibia sicherstellende Koppelungsachse (28) aufzunehmen.
